Europäisches Patentamt

(19) European Patent Office

Office européen des brevets

(11) **EP 0 848 832 B1**

(12) **EUROPÄISCHE PATENTSCHRIFT**

(45) Veröffentlichungstag und Bekanntmachung des Hinweises auf die Patenterteilung:
**13.03.2002 Patentblatt 2002/11**

(21) Anmeldenummer: **96931761.9**

(22) Anmeldetag: **03.09.1996**

(51) Int Cl.[7]: **G01V 3/08**, A61B 5/06

(86) Internationale Anmeldenummer:
**PCT/EP96/03857**

(87) Internationale Veröffentlichungsnummer:
**WO 97/09640 (13.03.1997 Gazette 1997/12)**

(54) **ANORDNUNG UND VERFAHREN ZUR BESTIMMUNG DER POSITION EINES MARKERS IN EINEM ORGANISCHEN HOHLRAUM**

METHOD AND ARRANGEMENT FOR DETERMINING THE POSITION OF A MARKER IN AN ORGANIC CAVITY

PROCEDE ET DISPOSITIF POUR DETERMINER LA POSITION D'UN MARQUEUR DANS UNE CAVITE ORGANIQUE

(84) Benannte Vertragsstaaten:
**ES FI FR GB GR IT PT SE**

(30) Priorität: **05.09.1995 DE 19532676**

(43) Veröffentlichungstag der Anmeldung:
**24.06.1998 Patentblatt 1998/26**

(73) Patentinhaber:
• **INSTITUT FÜR PHYSIKALISCHE HOCHTECHNOLOGIE E.V.**
**07745 Jena (DE)**
• **Asclepion-Meditec AG**
**07745 Jena (DE)**

(72) Erfinder:
• **ANDRÄ, Wilfried**
**D-07749 Jena (DE)**
• **EITNER, Klaus**
**D-07747 Jena (DE)**

• **HERGT, Rudolf**
**D-99510 Apolda (DE)**

(74) Vertreter: **Pfeiffer, Rolf-Gerd, Dipl.-Phys.**
**Patentanwaltsbüro Pfeiffer & Partner**
**Winzerlaer Strasse 10**
**07745 Jena (DE)**

(56) Entgegenhaltungen:
**DE-A- 3 940 260**

• **PATENT ABSTRACTS OF JAPAN vol. 009, no. 289 (P-405), 15.November 1985 & JP,A,60 128385 (YOKOKAWA HOKUSHIN DENKI KK), 9.Juli 1985,**
• **PATENT ABSTRACTS OF JAPAN vol. 009, no. 292 (P-406), 19.November 1985 & JP,A,60 129681 (YOKOKAWA HOKUSHIN DENKI KK;OTHERS: 01), 10.Juli 1985,**

**Beschreibung**

Die Erfindung betrifft eine Anordnung und ein Verfahren zur Bestimmung der Position eines Markers in einem organischen Hohlraum, insbesondere zur Ermittlung lokaler Passage-Geschwindigkeiten eines Markers durch den Magen-Darm-Trakt, insbesondere im Dünndarmbereich. Die Erfindung findet im Rahmen der medizinischen Diagnostik des Magen-Darm-Traktes Anwendung, ohne ein Diagnoseverfahren als solches zu betreffen.

Es sind medizinische Untersuchungen bekannt, bei denen eine wiederholte Bestimmung der lokalen Passage-Geschwindigkeit eines Markers durch den Magen-Darm-Trakt erforderlich sind. Das ist z.B. bei chronisch entzündlichen Darmerkrankungen, wie etwa Morbus Chron, funktioneller Magen-Darm-Erkrankungen und physiologischer Untersuchungen des Magen-Darm-Traktes der Fall.
Die übliche Diagnose-Technik, wie Röntgen unter Verwendung von Kontrastbrei, darf in solchen Fällen wegen der Strahlenbelastung nicht angewendet werden. Dasselbe gilt für szintigraphische Methoden.

Bekannte Techniken, bei denen die Strahlenbelastung vermieden wird, sind die Kernspin-Tomographie [M. Reiser, W. Semmler(Hrsg) "Magnetresonanztomographie", Springer-Verlag, Berlin/Heidelberg, 1992], die Sonographie [M. Amend, C. Jakobeit, L. Greiner, Verdauungskrankheiten 13 (1995), Heft 1, S. 21], der Einsatz von MetallDetektoren [K. Ewe, Therapiewoche 41 (1991), S. 77], der induktive Nachweis weichmagnetischer Tracer [Y. Benmair, B. Fischel, E. H. Frei, T. Gilat, The American Journal of Gastroenterology 68 (1977), S. 170] und das Orten von Dauermagnet-Markern [L. Trahms, R. Stehr, J. Wedemeyer, W. Weitschies, Biomedizinische Technik 35 (1990) S. 158].

Die Kernspin-Tomographie oder Magnet-Resonanz ist ein aufwendiges und teures Verfahren, das für häufig zu wiederholende Untersuchungen nicht geeignet und zur Ermittlung einer lokalen Passage-Geschwindigkeit, die einen zeitlichen Abstand aufeinanderfolgender Positionsbestimmungen von Markern in der Größenordnung von 10 s erforderlich macht, zu langsam ist [K. Fitzgerald, IEEE Spectrum 27 (1990) S. 52].

Sonographische Untersuchungen wurden bisher nicht zur Bestimmung der lokalen Passage-Geschwindigkeit, sondern nur zum Messen pauschaler Transitzeiten größerer Abschnitte des Magen-Darm-Traktes eingesetzt. [M. Amend, C. Jakobeit, L. Greiner, Verdauungskrankheiten 13 (1995), Heft 1, S. 21]. Der Grund liegt darin, daß Luftvolumina im Bauchraum von Ultraschall nicht durchdrungen werden, was zu einer fehlerhaften Positionsbestimmung von Markern führen würde. Diese Fehler könnten zwar herabgesetzt werden, wenn der Dann vollständig mit einer Flüssigkeit gefüllt würde; wegen der dadurch veränderten Peristaltik, ist aber ein gefüllter Darm zur Diagnose nicht geeignet.

Mit Metalldetektoren kann die Position von Metallteilchen bestimmt werden. Die laterale Genauigkeit bei der Positionsbestimmung nimmt jedoch mit dem Abstand von der Körperoberfläche ab und wird bei einem Abstand von > 10 cm schlechter als 1 cm [K. Ewe, Therapiewoche 41 (1991), S. 77]. Über die Genauigkeit einer Tiefenmessung wird in dieser Arbeit nichts berichtet. Da sie jedoch grundsätzlich schlechter als die laterale Genauigkeit ist, reicht dieses Verfahren für die Messung der lokalen Passage-Geschwindigkeit nicht aus.

Die Genauigkeit der Positionsbestimmung bei einer induktiven Messung weichmagnetischer Tracer genügt zur Untersuchung der zeitlichen Abnahme des Mageninhaltes an weichmagnetischem Brei mit einem Anfangsvolumen von über 100 cm$^3$ [Y. Benmair, B. Fischel, E. H. Frei, T. Gilat, The American Journal of Gastroenterology 68 (1977), S. 170]. Eine Messung der lokalen Passage-Geschwindigkeit im Darm ist damit jedoch nicht möglich, da das große Probenvolumen sich beim Durchlaufen des Darms unkontrolliert verteilt. Das Probenvolumen kann andrerseits aber auch nicht wesentlich verkleinert werden, da dann das vom Tracer stammende Sekundärmagnetfeld so klein wird, daß es selbst bei extrem guter Kompensation eines während der Messung angelegten Primärmagnetfeldes nicht mehr von dessen Restsignal getrennt werden kann.

Weiterhin ist bekannt, permanent magnetische Marker aufzumagnetisieren, bevor sie einem Patienten verabreicht werden [W. Weitschies, J. Wedemeyer, R. Stehr, L. Trahms, IEEE Trans. Biomed. Eng. 41 (1994) S. 192]. Die Positionsbestimmung der Marker aus ihrem Sekundärmagnetfeld wird hierbei allerdings durch Störfelder (z.B. das Erdmagnetfeld) so stark beeinflußt, daß die Messungen in einer Spezialkammer mit extremer magnetischer Abschirmung ausgeführt werden müssen. Selbst dann ist aber dieses Verfahren nicht für die Bestimmung der lokalen Passage-Geschwindigkeit im gesamten Magen-Darm-Trakt geeignet, da wegen der Transversal- und Rotationsbewegungen der Marker die Positionsbestimmung lediglich im Magen oder im Dickdarm möglich ist und selbst in diesen Bereichen mit einer relativ hohen Verweildauer des Markers nur mit einer ungenügenden Genauigkeit.

Der Erfindung liegt die Aufgabe zugrunde, eine Anordnung und ein Verfahren zur Positionsbestimmung von Markern in einem organischen Hohlraum, insbesondere im gesamten Magen-Darm-Trakt anzugeben, die eine Genauigkeit der Positionsbestimmung von besser als 1 cm in allen drei Koordinatenrichtungen gewährleistet und Zeiten für jede Positionsbestimmung von 10 s und darunter ermöglicht. Die durch die erfindungsgemäße Lösung gewonnenen Meßwerte für die jeweilige Markerposition sollen darüber hinaus einer räumlichen Darstellung zuführbar sein.

Die Aufgabe wird durch die Kennzeichen der unabhängigen Patentansprüche gelöst. Gemäß der Erfindung findet ein magnetisierbarer Marker, der solitär oder in einer viskosen Trägerlösung oder in Verbindung mit einem festen Trägermaterial (Kapsel) vorliegen kann, bestehend aus einem kugelsymmetrisch verteilten, isotropen halbharten Magnetmaterial, das eine mittelgroße Koerzitivfeldstärke, vorzugsweise im Bereich von $10^4$ bis $10^5$ A/m und eine große relative Remanenz, vorzugsweise > 0,8 besitzt, Anwendung. Bevorzugt besteht der Marker im wesentlichen aus $\gamma$-$Fe_2O_3$ und/oder $Fe_3O_4$, welches neben den geforderten magnetischen Eigenschaften zugleich nichttoxisch ist. Ein solcher Marker wird in den organischen Hohlraum eingebracht und einem Primärmagnetfeld einer elektrischen Spule, die eine impulsförmige Strombeaufschlagung erfährt, ausgesetzt. Der Spulendurchmesser soll dabei so groß bemessen sein, daß er mindestens ein 5faches des Markerdurchmessers beträgt. In jedem Fall ist die Spule so ausgebildet, daß sie ein rotationssymmetrisches nahezu homogenes Primärmagnetfeld in einem Gebiet der Größe von etwa $10 \cdot 10 \cdot 30 cm^3$ erzeugen läßt. Durch die impulsförmige Strombeaufschlagung genannter Spule wird der Marker während seiner Wanderung durch den organischen Hohlraum in kurzen Zeitabständen wiederholt aufmagnetisiert, wodurch sich das magnetische Moment des Markers jeweils parallel zum Primärmagnetfeld der Spule neu einstellt, so daß Rotationsbewegungen des Markers, als ggf. verfälschende Meßgröße, nicht mehr ins Gewicht fallen. Jeweils zwischen zwei Impulsen, wenn das Primärmagnetfeld der Spule auf Werte von vorzugsweise weniger als 10% des Maximalwertes abgeklungen ist, erfolgt mittels anisotroper Magnetfeldsensoren wenigstens eine Messung des Sekundärmagnetfeldes des Markers. Der zeitliche Abstand der Primärmagnetfeldmaxima ist den zu untersuchenden Magen-Darm-Trakt-Bereichen anpaßbar und wird in jedem Fall so kurz bemessen, daß störende Translations- oder Rotationsbewegungen des Markers nicht stattfinden oder durch Mittelwertbildungen mehrerer Messungen ausgeglichen werden können; vorzugsweise ist dieser zeitliche Abstand auf< 10 s festgelegt.

Bevorzugt wird das Primärmagnetfeld im Rahmen der Erfindung durch ein Paar koaxialer Spulen erzeugt, zwischen denen der Patientenkörper mit dem zu untersuchenden Bereich angeordnet wird. Der Abstand der Spule soll dem Patientenkörper angepaßt sein und beträgt vorzugsweise zwischen 30 cm und 40 cm. Die Richtung des Primärmagnetfeldes ist parallel zur gemeinsamen Achse beider Spulen und wird vorzugsweise zwischen zwei aufeinander folgenden Aufmagnetisierungen umgepolt. Der Maximalwert $H_p$ des Primärmagnetfeldes wird so gewählt, daß eine hinreichend hohe Remanenz des Markers erzeugt wird. Es ist beim gewählten Markermaterial vorteilhaft, ein $H_p$ von > $10^3$ A/m zu erzeugen. Nach oben ist $H_p$ allein durch den technischen Aufwand begrenzt und kann, z.B. bei Impulsfeldem, bis $10^5$ A/m betragen.

Das durch die Remanenz des Markers erzeugte Sekundärmagnetfeld $H_S$ kann in einem Abstand vom Markerzentrum, der wenigstens dreimal größer als der Markerdurchmesser ist, mit genügender Genauigkeit als Dipolfeld durch die Gleichungen

$$H_S = \frac{m}{R^3}\sqrt{(1+3\cos^2\vartheta)} \qquad (1)$$

$$\tan \Phi = 0,5 \tan \vartheta \qquad (2)$$

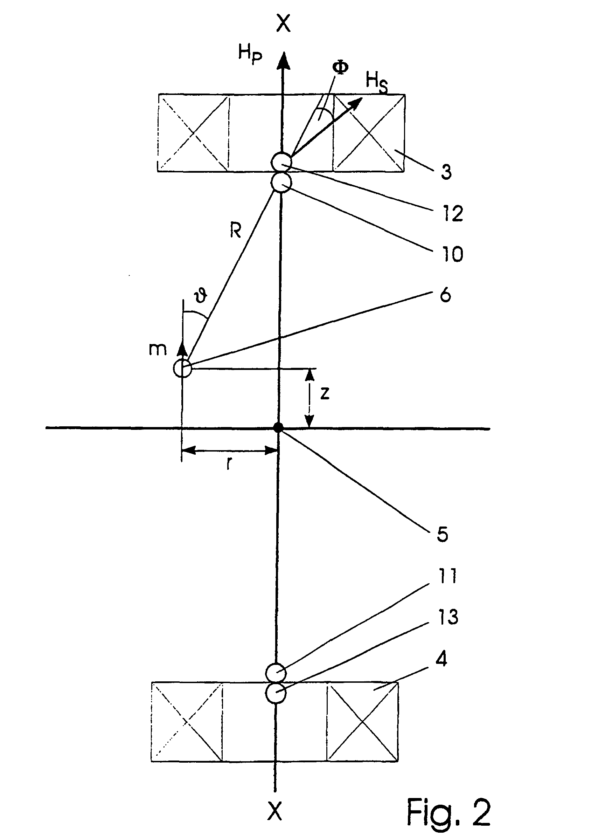

beschrieben werden, wobei m das magnetische Moment des Markers, R den Abstand zwischen Marker-Zentrum und Ort des Sekundärmagnetfeldes, sowie $\Phi$ und $\vartheta$ die in Figur 2 definierten Winkel bedeuten. Die Komponenten des Sekundärmagnetfeldes parallel $H_S^{\parallel}$ und senkrecht $H_S^{\perp}$ zur Remanenzrichtung werden durch die Gleichungen

$$H_S^{\parallel} = H_S \cos (\vartheta + \Phi) \qquad (3)$$

$$H_S^{\perp} = H_S \sin(\vartheta + \Phi) \qquad (4)$$

beschrieben. Diese Komponenten lassen sich mittels geeigneter Magnetfeldsensoren, vorzugsweise anisotroper, magnetoresistiver Dünnschichtsensoren, wie sie derzeit verfügbar sind, getrennt vermessen. Auf der durch die m-Richtung bestimmten Achse des Sekundärmagnetfeldes gilt $\vartheta = \Phi = 0$. Bei vorgegebenen Werten von m und R hat daher der Betrag von $H_S^{\parallel}$ ein Maximum, während gleichzeitig $H_S^{\perp}$ Null ist. Sobald der Marker sich auf der Spulenachse befindet, zeigt demnach ein auf dieser Spulenachse angeordneter Magnetfeldsensor fiir $H_S^{\perp}$ nur ein eventuell vorhandenes Störfeld an. Bei Abweichung des Markers von der Spulenachse liefert der Magnetfeldsensor jedoch ein Zusatzsignal, dessen Vorzeichen mit der Polarität der Marker-Remanenz, also mit der Polarität des Primärmagnetfeldes, wechselt. Dieses frequenzselektiv verstärkbare Signal dieses Magnetfeldsensors zeigt daher an, ob sich der Marker auf der Spulenachse befindet. Bei kleinen Winkeln $\vartheta$ und $\Phi$ ist $H_S^{\perp}$ eine monotone Funktion des Abstandes r zwischen dem Markerzentrum und der Spulenachse (und des darauf fixierten wenigstens einen Magnetfeldsensors) und wird Null bei r = 0. Durch Verschieben der Feldachse (und des darauf fixierten Magnetfeldsensors) gegenüber dem Marker ist daher das Signal auf Null abgleichbar, weshalb gemäß der Erfindung die Spulenachse ins Markerzentrum nachgeführt wird. Zur Erhöhung der Genauigkeit kann ein zweiter, auf der Spulenachse symmetrisch angeordneter und fixierter Magnetfeldsensor zur Bestimmung von $H_S^{\perp}$ angeordnet sein, dessen Signal mit umgekehrtem Vorzeichen dem Signal des ersten Magnetfeldsensors addiert wird. Ebenso können mehrere solcher Magnetfeldsensoren radial um die Spulenachse axialsymmetrisch verteilt angeordnet und in o.g. Sinn elektrisch verschaltet sein. Sobald der Marker sich auf der Spulenachse befindet ($\vartheta = \Phi = 0$), ist bei vorgegebenem Wert des magnetischen Moments m der Betrag von $H_S$ gemäß Gleichung (1) eine eindeutige Funktion von R. Im Spezialfall, unter Verwendung zweier koaxial zueinander angeordneter gleicher Spulen und der Position des Marker genau in der Mitte zwischen diesen Spulen und dem Einsatz je eines zur jeweiligen Spule und zur gemeinsamen Spulenachse gleich positionierten $H_S^{\parallel}$-Magnetfeldsensors, ist $H_S$ am Ort dieser beiden Magnetfeldsensoren gleich groß. Falls beide Sensoren die gleiche Empfindlichkeit besitzen, ist die Differenz dieser Signale Null. Weicht die Markerposition auf der Spulenachse um einen Abstand z von genannter Mittenstellung ab, dann ist das Differenzsignal eine monotone Funktion von z und, bei bekanntem m, ein Maß für die Abweichung des Markers in z-Richtung. Der Wert von m kann in einem gesonderten Meßschritt bestimmt werden, bevor und/oder nachdem der Marker dem Patienten verabreicht wird bzw. wurde. Die Genauigkeit der Messung in z-Richtung kann analog zur Bestimmung in radialer Richtung durch weitere auf der Spulenachse angeordnete und elektrisch entsprechend verschaltete $H_S^{\parallel}$-Magnetfeldsensoren erhöht werden.

Die jeweils aktuelle Position des Markers innerhalb des organischen Hohlraums ist somit zum einen durch die Position der Spulenachse, zu deren Registrierung geeignete Mittel vorgesehen sind, nach erfolgtem Null-Abgleich des $H_S^{\perp}$-Signals mittels geeigneter Verschiebemittel und zum anderen durch die Größe des $H_S^{\parallel}$-Signals vollständig bestimmt.

Um die Markerposition relativ zum Patientenkörper zu bestimmen, etwa zur Erst- bzw. Zwischenausrichtung des Patienten, werden Bezugs-Marker in Form von kleinen Spulen bzw. Magneten, auf dem Patientenkörper an definierten Stellen befestigt, deren Position ebenfalls vermessen wird.

Die Bestimmung der lokalen Passage-Geschwindigkeit des Markers ergibt sich in leichter Weise als Quotient aus dem jeweiligen Abstand zweier Meßpunkte zwischen je zwei Stromimpulsbeaufschlagungen der Spule(n) und der Zeitdifferenz zwischen diesen benachbarten Punkten.

Die Erfindung soll im nachstehenden anhand eines Ausführungsbeispiels und vier schematischer Zeichnungen näher erläutert werden. Es zeigen:

Fig. 1 die Positionierung eines Patienten innerhalb einer möglichen Anordnung gemäß der Erfindung,
Fig. 2 eine schematische Darstellung der Verhältnisse bei Abweichung eines Markers aus der Spulenachse,
Fig. 3 eine erweiterte Darstellung einer Gesamtanordnung gemäß Fig. 1 und
Fig. 4 ein Diagramm zur Verdeutlichung, wie die Spulen erfindungsgemäß mit Stromimpulsen beaufschlagt werden.

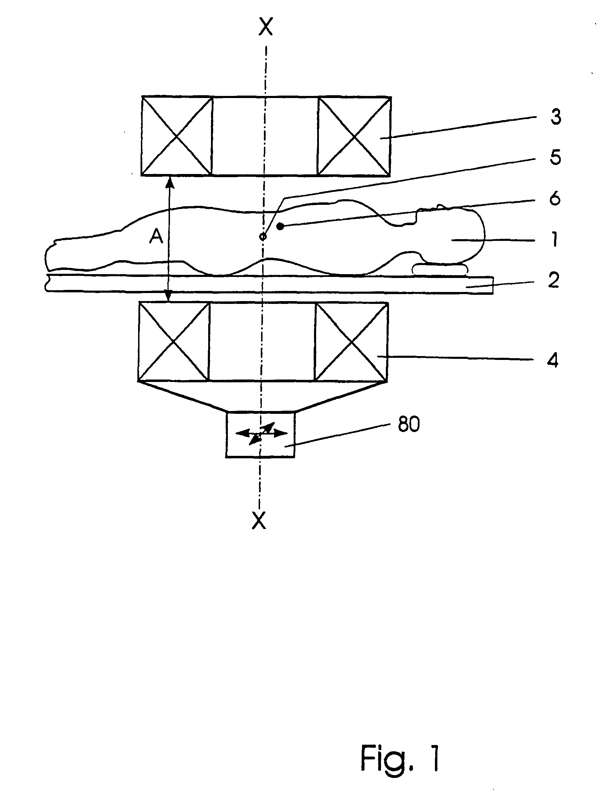

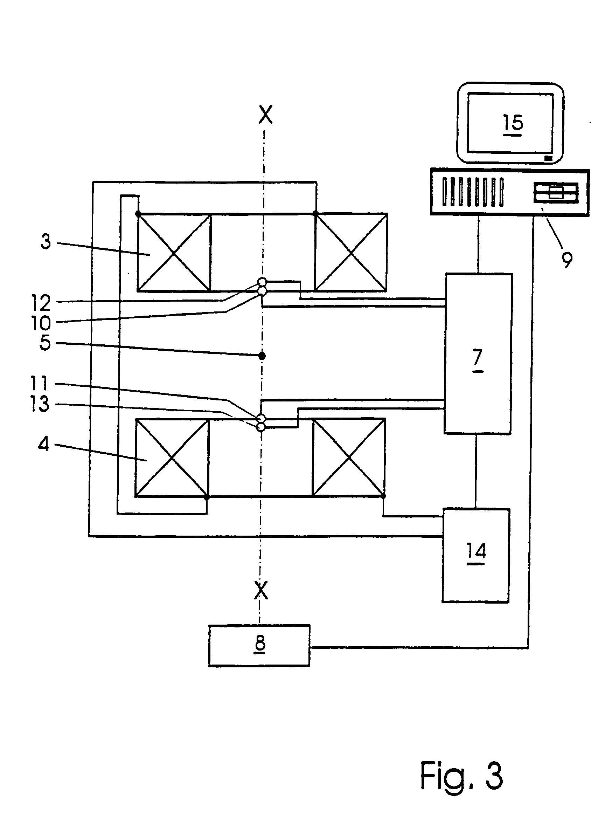

In Figur 1 liegt ein Patient 1, dessen Magen-Darm-Trakt untersucht werden soll, auf einer nichtmetallischen Auflage 2. Zwei identische Spulen 3 und 4 sind mit ihren Spulenachsen auf einer gemeinsamen Achse X-X in einer lichten Weite A in der Größenordnung von 30 cm beabstandet angeordnet. Mittels eines in Fig. 3 näher bezeichneten Generators 14 werden die Spulen 3, 4 mit impulsförmigen Strömen beaufschlagt. Im geometrischen Zentrum 5 wird dabei ein maximales Primärmagnetfeld bspw. mit einer Stärke von 20000 A/m, je nach gewählter Stromimpulsfrequenz bspw. in einem Zeitabstand von 1 s erzeugt. Dem Patienten 1 wurde oral ein Marker 6 bestehend aus $\gamma$-$Fe_2O_3$ verabreicht, dessen Durchmesser ca. 8 mm beträgt. Nach jedem Aufmagnetisieren in genanntem Primärmagnetfeld besitzt der Marker 6 ein remanentes magnetisches Moment m von ca. $7 \cdot 10^{-5}$ $Am^2$.

In Figur 2 ist schematisch angedeutet, wie die Spulen 3, 4 jeweils mit anisotropen magnetoresistiven Magnetfeldsensoren 10, 11, 12, 13 in eine starre Verbindung gebracht sind. Dabei sind die Magnetfeldsensoren 10, 11 so ausgelegt, daß sie der ausschließlichen Detektion einer parallel zur Achse X-X verlaufenden Komponente des vom Marker ausgehenden Sekundärmagnetfeldes und die Magnetfeldsensoren 12, 13 einer senkrecht dazu verlaufenden Radialkomponente genannten Sekundärmagnetfeldes dienen. Befindet sich der Marker 6 im Zentrum der gemeinsamen Spulenachse X-X, beträgt die Magnetfeldkomponente $H_S^{\parallel}$, entsprechend obiger Maßgaben, am Ort der Magnetfeldsensoren 10, 11 ca. $4 \cdot 10^{-2}$ A/m, während die dazu senkrecht verlaufende Magnetfeldkomponente $H_S^{\perp}$ Null ist. In Figur 2 sind nunmehr die Verhältnisse angedeutet, die entstehen, wenn der Marker aus der Zentrallage 5 radial bspw. um r = 1 cm relativ zur Feldachse verschoben ist. Hier beträgt dann bspw. die Radialkomponente des Sekundärmagnetfeldes am Ort der Radialsensoren 12, 13 etwa $4 \cdot 10^{-3}$ A/m. Nach einer Axialverschiebung des Markers um die Strecke z = 1 cm, wie dargestellt, beträgt die Axialkomponente des Sekundärmagnetfeldes am Ort des Sensors 10 etwa $5,2 \cdot 10^{-2}$ A/m, wohingegen sie am Ort des Sensors 11 etwa nur $3,5 \cdot 10^{-2}$ A/m aufweist.

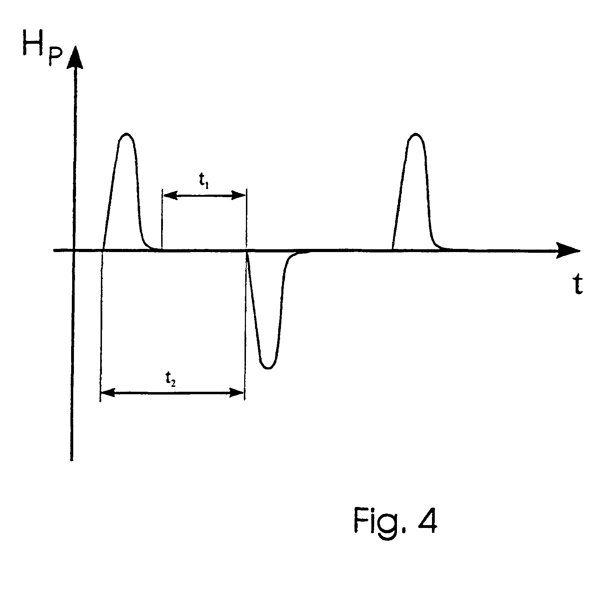

Anisotrope Magnetfeldsensoren können Signalspannungen von $10^{-4}$ V bei einer Feldstärke von 1 A/m liefern. Werden die Sensoren jeweils in den erfindungsgemäß vorgesehenen Pausen $t_1$ (vgl. Fig. 4) zwischen zwei Primärfeldimpulsen mit dem zeitlichen Abstand $t_2$ getriggert eingeschaltet, dann liefern die in Reihe geschalteten Radialsensoren 12, 13 nach einer radialen Markerverschiebung von r = 1 cm eine impulsförmige Wechselspannung mit einer Amplitude von 0,4 $\mu$V, die mit einem frequenzselektiven Verstärker 7 leicht auf 40 $\mu$V verstärkbar ist. Die im Beispiel gemeinsame Spulenachse X-X wird nun gemäß der Erfindung mit Hilfe von Mitteln zur relativen Nachführung, die am einfachsten in Form eines mit den Spulen 3 un 4 in Verbindung stehenden Kreuztisches 80 (vgl. Fig. 1) ausgebildet sein können, solange verschoben, bis dieses Signal verschwunden ist. Ist dies der Fall, befindet sich die Spulenachse wieder im Markerzentrum. Ihre diesbezüglich eingenommene Lage wird mit einem in Fig. 3 angedeuteten Ortssensor 8 erfaßt und einer Speicher- und Auswerteeinheit 9 zugeführt.

Nach einer Axialverschiebung des Markers 6 aus dem Zentrum 5 um z = 1 cm, im Beispiel nach oben, werden von den beiden Axialsensoren 10, 11 impulsförmige Wechselspannungen mit Amplituden von ca. 5,2 µV (am Sensor 10) bzw. ca. 3,5 µV (am Sensor 11) erzeugt. Die Differenz von 1,7 µV kann ebenfalls mit dem frequenzselektiven Verstärker 7 auf etwa 170 µV verstärkt werden. Diese der jeweiligen vertikalen Markerposition zugeordneten Signale werden ebenfalls der Speicher- und Auswerteeinheit 9 zugeführt. Somit steht für jede aktuelle Markerposition die erforderliche Anzahl von Meßwerten zur Verfügung, die seine aktuelle räumliche Lage im Magen-Darm-Trakt eindeutig beschreibt. Die auf die beschriebene Weise nacheinander gewonnenen Markerpositionen bilden eine Punktkette, die den Weg des Markers repräsentiert. Der Quotient aus dem Abstand benachbarter Punkte und dem Zeitintervall zwischen diesen Punkten steht dabei als Maß für die zu ermittelnde lokale Passage-Geschwindigkeit des Markers 6. All die gewonnenen Meßdaten lassen sich nach entsprechender Auswertung und Kalibrierung während oder nach der Untersuchung der Markerbewegung im Patienten auf einem Monitor 15 dreidimensional zur Anzeige bringen.

Die im Rahmen des Ausführungsbeispiels dargestellte Verwendung zweier Spulen 3, 4 ist eine besonders vorteilhafte Ausführungsform, beschränkt die Erfindung jedoch nicht darauf. Ebenso sind Ausführungen mit nur einer Spule oder mit mehr als zwei geeignet angeordneten Spulen denkbar.

Beim erfindungsgemäßen Verfahren ist der Patient keinen Strahlenbelastungen ausgesetzt und die Messung kann beliebig oft wiederholt werden. Besonders vorteilhaft bei der erfindungsgemäßen Lösung ist, daß das Erdmagnetfeld bzw. das lokale örtliche Störfeld keine Auswirkungen auf das gesuchte Meßergebnis hat und so keinerlei aufwendige Abschirmmaßnahmen erforderlich sind.

Bezugszeichenliste

| | |
|---|---|
| 1 - | Patient |
| 2 - | Auflage |
| 3,4 - | Spule |
| 5 - | geometrisches Zentrum |
| 6 - | Marker |
| 7 - | frequenzselektiver Verstärker |
| 8 - | Ortssensor |
| 80 - | Mittel zur Nachführung |
| 9 - | Speicher- und Auswerteeinheit |
| 10, 11 - | Axialsensoren |
| 12, 13 - | Radialsensoren |
| 14 - | Generator |
| 15 - | Monitor |
| A - | lichte Weite |
| $t_1$ - | Meßintervall |
| $t_2$ - | Intervall der Stromimpulsfolge |
| X-X - | Achse |
| $\vartheta, \Phi$ - | Winkel |

**Patentansprüche**

1. Anordnung zur Bestimmung der Position eines Markers in einem organischen Hohlraum, bei der ein magnetisierbarer Marker und Mittel zur Positionsbestimmung des Markers zum Einsatz gelangen, **dadurch gekennzeichnet, daß**

   a) ein im organischen Hohlraum bewegbarer, magnetisierbarer Marker (6), bestehend aus einem halbharten Magnetmaterial mit einer großen relativen Remanenz und mittelgroßer Koerzitivfeldstärke,
   b) wenigstens eine elektrische Spule (3 oder 4), der ein Spulendurchmesser gegeben ist, der mindestens ein 5faches des Markerdurchmessers beträgt,
   c) Mittel (14) zur impulsförmigen Strombeaufschlagung genannter Spule,
   d) anisotrope Magnetfeldsensoren (10, 11; 12, 13), die axialsymmetrisch zur Spulenachse (X-X) genannter Spule (3 oder 4) und in einer starren geometrischen Verbindung zur Spule angeordnet sind,
   e) Mittel (80) zur Nachführung der Spulenachse in bezug auf die aktuelle Markerposition und Mittel (8) zur Positionsbestimmung der Spulenachse (X-X) und
   f) Mittel (7; 9) zur Signalerfassung, Speicherung und Auswertung der Signale der anisotropen Magnetfeldsensoren (10, 11; 12, 13) und der Mittel (8) zur Positionsbestimmung

   vorgesehen sind.

2. Anordnung gemäß Anspruch 1, **dadurch gekennzeichnet, daß** die wenigstens eine Spule (3 oder 4) so ausgebildet ist, daß sie ein rotationssymmetrisches, in einem Meßbereich in der Größenordnung von $10 \cdot 10 \cdot 30$ cm$^3$, nahezu homogenes magnetisches Primärfeld erzeugen läßt.

3. Anordnung gemäß Anspruch 1 und 2, **dadurch gekennzeichnet, daß** bei Einsatz zweier Spulen (3, 4) diese zueinander koaxial und voneinander in einer lichten Weite (A) angeordnet sind, die die Aufnahme genannten organischen Hohlraums zuläßt.

**4.** Anordnung gemäß Anspruch 1., **dadurch gekennzeichnet, daß** der Marker (6) kugelsymmetrisch ausgebildet ist und aus einem isotropen Magnetmaterial besteht, das eine Koerzitivfeldstärke im Bereich von $10^4$ bis $10^5$ A/m und eine relative Remanenz von vorzugsweise > 0,8 besitzt.

**5.** Anordnung gemäß Anspruch 4, **dadurch gekennzeichnet, daß** der Marker im wesentlichen aus $\gamma$-$Fe_2O_3$ und/oder $Fe_3O_4$ gefertigt ist.

**6.** Anordnung gemäß Anspruch 1, **dadurch gekennzeichnet, daß** für die anisotropen Magnetfeldsensoren (10, 11; 12, 13) magnetoresistive Dünnschichtsensoren gewählt sind.

**7.** Anordnung gemäß Anspruch 1 und 6, **dadurch gekennzeichnet, daß** die anisotropen Magnetfeldsensoren so angeordnet sind, daß

- wenigstens einer (10 oder 11) auf der Spulenachse zur Bestimmung der parallel zur Spulenachse (X-X) verlaufenden Magnetfeldkomponente des magnetischen Sekundärfeldes des Markers (6)
- und wenigstens einer (12 oder 13) zur Bestimmung einer senkrecht zur Spulenachse (X-X) verlaufenden radialen Magnetfeldkomponente des magnetischen Sekundärfeldes des Markers (6)

eingesetzt ist.

**8.** Anordnung gemäß Anspruch 1 und 7, **dadurch gekennzeichnet, daß** bei Einsatz mehrerer anisotroper Magnetfeldsensoren zur Bestimmung der senkrecht zur Spulenachse verlaufenden radialen Magnetfeldkomponente des magnetischen Sekundärfeldes des Markers (6) diese axialsymmetrisch zur Spulenachse (X-X) und in einer senkrecht zur Spulenachse verlaufenden Ebene angeordnet sind.

**9.** Anordnung gemäß Anspruch 1, 6, 7 oder 8, **dadurch gekennzeichnet, daß** die anisotropen Magnetfeldsensoren (10, 11; 12, 13) in einem Abstand zum Marker (6) angeordnet sind, der mindestens ein Dreifaches des Markerdurchmessers beträgt.

**10.** Verfahren zur Bestimmung der Position eines Markers in einem organischen Hohlraum unter Verwendung einer Anordnung gemäß einem der vorstehenden Ansprüche, **dadurch gekennzeichnet, daß**

a) die wenigstens eine Spule impulsförmig in einem zeitlichen Abstand mit im Vorzeichen jeweils wechselnden Strom beaufschlagt wird,
b) zwischen zwei Stromimpulsen, wenn das durch sie erzeugte Primärmagnetfeld der Spule auf hinreichend kleine Werte abgeklungen ist, wenigstens einmal das von einem Marker stammende Sekundärmagnetfeld mittels anisotroper Magnetfeldsensoren parallel und senkrecht zur Spulenachse getrennt vermessen wird,
c) die Spulenanordnung relativ zum Marker solange verschoben wird, bis die Magnetfeldsensoren, die der Bestimmung der radialen Sekundärmagnetfeldkomponente des Markers dienen, ein Nullsignal liefern
d) und diese zugehörige Position der Spulenachse, als auch die zugehörigen Meßwerte, die die Magnetfeldsensoren, die der Erfassung der parallel zur Spulenachse verlaufenden Sekundärmagnetfeldkomponente des Markers dienen, abgespeichert und einer Anzeige zugeführt werden.

**11.** Verfahren nach Anspruch 10, **dadurch gekennzeichnet, daß** die Signale der anisotropen Magnetfeldsensoren, welche durch das Sekundärmagnetfeld des Markers erzeugt werden, zu einem Zeitpunkt getriggert ausgelesen werden, wenn das durch die Spule erzeugte Primärmagnetfeld zwischen zwei Stromimpulsen auf weniger als 10% seines Maximalwertes abgeklungen ist.

**12.** Verfahren nach Anspruch 10 oder 11, **dadurch gekennzeichnet, daß** zwischen zwei Stromimpulsbeaufschlagungen der Spule mehrere Messungen des Sekundärmagnetfeldes und eine Mittelung der Meßwerte vorgenommen werden.

**13.** Verfahren nach einem der Ansprüche 10 bis 12, **dadurch gekennzeichnet, daß** der zeitliche Abstand zweier Stromimpulsbeaufschlagungen unterhalb von 10 s festgelegt wird.

**Claims**

**1.** An arrangement for determining the position of a marker in an organic cavity comprising a magnetizable marker and means for determining the position of said marker, **characterized in** providing

a) in said organic cavity a moveable and magnetizable marker (6) made of a semi-hard magnetic material of a comparatively great relative residual magnetism and a medium-size coercive field strength,
b) at least one electric coil (3 or 4) having a coil diameter of at least fivefold the diameter of said marker,
c) means (14) of applying a pulse-shaped current across said coil,

d) anisotropic magnetic field sensors (10, 11; 12, 13) being arranged in axial symmetry to a coil axis (X-X) of said coil (3 or 4) and being in a non-displaceable geometric connection to said coil,

e) means (80) for tracking the coil axis relative to the actual marker position, and means (8) for detecting the position of the coil axis (X-X), and

f) means (7, 9) for signal detection, for storing and evaluating the signals derived from said anisotropic magnetic field sensors (10, 11, 12, 13), and means for position detection.

2. An arrangement as claimed in claim 1, **characterized in that** at least one coil (3 or 4) is adapted to generate an axially symmetrical and, over a measuring range of about $10 \cdot 10 \cdot 30$ cm$^3$, substantially homogeneous primary magnetic field.

3. An arrangement as claimed in claims 1 and 2, **characterized in that**, when two coils (3, 4) are employed, the latter are arranged coaxially to one another and spaced apart by an inner width (A) which permits to place said organic cavity in between.

4. An arrangement as claimed in claim 1, **characterized in that** the marker (6) is spherosymmetrically embodied and made of an isotropic magnetic material exhibiting a coercive field strength over a range of from $10^4$ up to $10^5$ A/m, and a relative residual magnetism of preferably > 0.8.

5. An arrangement as claimed in claim 4, **characterized in that** the marker substantially is made of $\gamma\text{-Fe}_2\text{O}_3$ and/or $\text{Fe}_3\text{O}_4$.

6. An arrangement as claimed in claim 1, **characterized in that** magnetoresistive thin layer sensors are selected for said anisotropic magnetic field sensors (10, 11; 12, 13).

7. An arrangement as claimed in claims 1 and 6, **characterized in that** said anisotropic magnetic field sensors are arranged that

- at least one of said sensors (10 or 11) is positioned along the coil axis to detect the magnetic field component of the secondary magnetic field of the marker (6) in parallel to the coil axis (X-X), and
- at least one of said sensors (12 or 13) is positioned to detect the radial magnetic field component of the secondary magnetic field of the marker (6) which is at right angles to the coil axis (X-X).

8. An arrangement as claimed in claims 1 and 7, **characterized in that**, when employing a plurality of anisotropic magnetic field sensors to detect the radial magnetic field component of the secondary magnetic field of the marker (6) which is at right angles to the coil axis, said plurality of anisotropic magnetic field sensors is arranged in axial symmetry to the coil axis (X-X) in a plane at right angles to the coil axis.

9. An arrangement as claimed in claims 1, 6, 7 or 8, **characterized in that** said anisotropic magnetic field sensors (10, 11; 12, 13) are arranged in spaced relation to the marker (6), which relation is at least three-times the marker diameter.

10. Method for determining the position of a marker in an organic cavity under use of the arrangement as disclosed in one of the above claims, **characterized in that**

a) a pulse-shaped current, with signs alternating at respective time intervals, being applied across at least one coil,

b) the secondary magnetic field originating from a marker is at least one time measured separately, parallel as well as at right angles to the coil axis, by means of anisotropic magnetic field sensors between two current pulses, when the primary magnetic field of the coil produced by said pulses decayed to sufficiently low values,

c) the coil arrangement is displaced relative to the marker until the magnetic field sensors, which detect the radial secondary magnetic field component of the marker, deliver a zero-signal, and

d) the associated position of the coil axis and the respective measuring values, detected by the magnetic field sensors which are adapted to detect the secondary magnetic field component of the marker in parallel to the coil axis, are stored and fed into a display.

11. Method as claimed in claim 10, **characterized in that** the signals of the anisotropic magnetic field sensors generated by the secondary magnetic field of the marker are triggered and read at a point of time when the primary magnetic field produced by the coil has decayed to less than 10% of its maximum between two current pulses.

12. Method as claimed in claim 10 or 11, **characterized in that** a plurality of measurements of the secondary magnetic field is carried out between two current pulses applied across the coil, and **in that** the measuring values are submitted to a mean value formation.

13. Method as claimed in any of the preceding claims 10 to 12, **characterized in that** the time interval between two current pulses is set to < 10 s.

**Revendications**

**1.** Le dispositif pour la détermination de la position d'un marqueur dans une cavité organique à l'aide d'un marqueur magnétisable et de moyens permettant la détermination de la position du marqueur est **caractérisé en ce que** la conception prévoit :

a) un marqueur magnétisable (6) qui se déplace dans une cavité organique, composé d'un matériel aimanté mi-dur qui est **caractérisé par** une rémanence relativement importante et une intensité coercitive moyenne du champ magnétique,
b) au moins une bobine électrique (3 ou 4) dont le diamètre est cinq fois plus grand que le diamètre du marqueur,
c) dispositif (14) destiné à l'alimentation en courant par impulsions de la bobine mentionnée,
d) détecteurs anisotropes du champ magnétique (10; 11; 12; 13), disposés de manière axisymétrique par rapport à l'axe X - X de la bobine (3 ou 4) et dans une construction géométrique rigide par rapport à la bobine ,
e) dispositif (80) destiné à déplacer l'axe de la bobine en fonction de la position concrète du marqueur à un moment donné et dispositif (8) destiné à la détermination de la position de l'axe de la bobine (X-X) et
f) dispositif (7; 9) destiné à la saisie, la mémorisation et l'interprétation des signaux émis par les détecteurs anisotropes du champs magnétique (10, 11 ; 12, 13) et dispositif (8) destiné à la détermination de la position.

**2.** Le dispositif magnétique suivant la revendication 1 est **caractérisé en ce qu'**au moins une des bobines ( 3 ou 4) est conçue de sorte à être en mesure de générer un champ primaire homogène à symétrie de révolution pour une plage de mesure de 10 · 10 · 30 cm$^3$.

**3.** Le dispositif suivant les revendications 1 et 2 est **caractérisé en ce que** dans le cas où l'on utilise deux bobines ( 3, 4) celles-ci sont placées de manière coaxiale, l'une par rapport à l'autre, dans une distance intérieure (A) qui permet la prise de vue de la cavité organique susmentionnée.

**4.** Le dispositif suivant la revendication 1 est **caractérisée en ce que** le marqueur (6) est conçu en symétrie sphérique et composé d'un matériel magnétique isotrope dont l'intensité du champ coercitif se situe dans la plage de 10$^4$ à 10$^5$ A/m et se distingue par une rémanence relative étant de préférence > 0,8.

**5.** Le dispositif suivant la revendication 4 est **caractérisé en ce que** le marqueur est fait essentiellement en γ-Fe$_2$O$_3$ et/ou en Fe$_3$O.

**6.** La disposition suivant la revendication 1 est **caractérisée en ce que** les détecteurs du champ magnétique anisotrope ( 10; 11; 12; 113) sont des détecteurs à base de couches minces qui se distinguent par une magnétorésistivité.

**7.** Le dospositif suivant les revendications 1 et 6 est **caractérisé en ce que** les détecteurs anisotropes du champ magnétique sont montés de sorte que

- au moins un des détecteurs ( 10 ou 11) placé sur l'axe de la bobine soit destiné à la détermination de la composante du champ magnétique secondaire du marqueur (6), orientée dans le sens parallèle par rapport à l'axe de la bobine ( X- X) et
- au moins un des détecteurs ( 12 ou 13) soit destiné à la détermination de la composante radiale du champ magnétique secondaire du marqueur (6), orientée dans le sens perpendiculaire par rapport à l'axe de la bobine (X - X).

**8.** Le dispositif suivant les revendications 1 et 7 est **caractérisé en ce que**, dans le cas où plusieurs détecteurs anisotropes du champ magnétique sont utilisés pour déterminer la composante radiale du champ magnétique secondaire du marqueur (6), orientée dans le sens perpendiculaire par rapport à l'axe de la bobine, ceux- ci sont placés de manière axisymétrique par rapport à l'axe de la bobine (X-X) et dans un plan en position perpendiculaire par rapport à l'axe de la bobine.

**9.** Le dispositif suivant les revendications 1, 6, 7 ou 8 est **caractérisé en ce que** les détecteurs anisotropes du champ magnétique (10; 11 ; 12; 13) sont placés de sorte que la différence qui les sépare du marqueur (6) reste au moins trois fois plus grande que le diamètre du marqueur.

**10.** Le procédé destiné à déterminer la position du marqueur dans une cavité organique en appliquant le dispositif tel qu'il est **caractérisé** dans les revendications précédentes, est lui-même **caractérisé en ce que**

a) au moins une bobine est alimentée en courant par impulsions dans un intervalle déterminé et en alternant son signe,
b) entre deux impulsions de courant, au moment où l'intensité du champ magnétique primaire de la bobine qu'elles ont généré, a suffisamment diminué, le champ magnétique secondaire généré par un marqueur, est mesuré individuellement à l'aide de détecteurs anisotropes du champ magnétique dans le sens parallèle et dans le sens perpendiculaire par rapport à l'axe,

c) la disposition de la bobine par rapport au marqueur est modifiée jusqu'à ce que les détecteurs du champ magnétique destinés à déterminer la composante radiale du champ magnétique secondaire du marqueur émettent un signal égal à zéro,

d) la position de l'axe de la bobine ainsi que les valeurs mesurées dans cette position par les détecteurs du champ magnétique destiné à saisir la composante du champ magnétique secondaire du marqueur, orientée dans le sens parallèle par rapport à l'axe de la bobine sont mémorisées et affichées.

**11.** Le procédé selon la revendication 10 est **caractérisé en ce que** les signaux générés par le champ magnétique secondaire du marqueur et émis par les détecteurs anisotropes du champ magnétique sont lus et enregistrés au moment où dans la période entre deux impulsions, l'intensité du champ magnétique primaire généré par la bobine a diminué de sorte qu'elle ne représente que 10% de sa valeur maxi.

**12.** Le procédé selon la revendication 10 ou 11 est **caractérisé en ce que**, dans la période entre deux alimentations en courant par impulsions de la bobine, l'intensité du champ magnétique secondaire est mesuré à plusieurs reprises et les valeurs mesurées sont communiquées.

**13.** Le procédé selon les revendications 10 à 12 est **caractérisé en ce que** l'intervalle entre deux alimentations en courant par impulsion doit être inférieur à 10 s.

X

3

5

6

A

1

2

4

80

Fig. 1

Fig. 2

Fig. 3

Fig. 4